# NOUVEAU FASCICULE DE BREVET EUROPEEN

(11) **EP 1 627 667 B2**
(45) Date de publication et mention de la décision concernant l'opposition: **22.05.2013**
(45) Mention de la délivrance du brevet: 01.10.2008
(21) Numéro de dépôt: 05291631.9
(22) Date de dépôt: 01.08.2005
(51) Int. Cl.: A61Q 5/12, A61K 8/06, A61K 8/92, A61K 8/81, A61K 8/37, A61K 8/41, A61K 8/45, A61K 8/34, A61K 8/39, A61K 8/60

(54) **Emulsion eau-dan-huile, comprenant une huile non-volatile non-siliconée, des tensioactifs cationique et non ioniques, et un alkylmonoglycoside ou alkylpolyglycoside**
Wasser-in-Öl Emulsion mit einem nicht-flüchtigen Öl ohne Silicongruppen, kationischen und nichtionischen Detergentien, sowie einem Alkylmonoglycosid oder Alkylpolyglycosid
Water-in-oil emulsion comprising a non-volatile non-silicon oil, cationic and non-ionic surfactants, and an alkyl monoglycoside or an alkyl polyglycoside

(30) Priorité: 02.08.2004 FR 0408537
(43) Date de publication de la demande: 22.02.2006
(73) Titulaire: L'Oréal SA, 75008 Paris (FR)
(72) Inventeur: Fack, Géraldine, 92300 Levallois-Perret (FR); Pourille-Grethen, Chrystel, 92110 Clichy (FR)
(74) Mandataire: Casalonga, Axel

(56) Documents cités:
- EP-A1- 1 093 801
- WO-A-01/08648
- WO-A1-94/06408
- WO-A1-01//45649
- WO-A1-04/030641
- DE-A1- 441 157
- DE-A1- 4 301 820
- DE-A1- 4 405 510
- DE-A1- 19 710 155
- DE-U1- 29 903 136
- FR-A- 2 847 831
- US-A- 6 156 076
- KARHE J ET AL: "ALKYLPOLYGLYCOSIDE EIN NEUES KONZEPT FUR PFLEGE UND VERTRAGLICHKEIT IN DER KOSMETIK" SOFW-JOURNAL SEIFEN, OELE, FETTE, WACHSE, VERLAG FUR CHEMISCHE INDUSTRIE, H. ZIOLKOWSKY K.G. AUGSBURG, DE, vol. 121, no. 8, 1 juillet 1995 (1995-07-01), pages 598,600-601,604, XP000514057 ISSN: 0942-7694
- ANONYMOUS: "New cosmetic formulations" RESEARCH DISCLOSURE, KENNETH MASON PUBLICATIONS, HAMPSHIRE, GB, vol. 443, no. 43, mars 2001 (2001-03), XP007127759 ISSN: 0374-4353
- FEY, OTTE: 'Wörterbuch der Kosmetik', vol. 4, 1995, WISSENSCHAFTLICHE VERLAGSGESELLSCHAFT MBH, STUTTGART pages 151,115 3 - 124
- PROF. DR. FALBE, JÜRGEN ET AL.: 'Römpp Chemie Lexikon', 1995, GEORG THIEME VERLAG, STUTTGART, NEW YORK pages 1158 - 1159
- PROF. DR. FALBE JÜRGEN ET AL.: 'Römpp Chemie Lexikon', vol. 3, 1995, GEORG THIEME VERLAG, STUTTGART, NEW YORK pages 1812 - 1813

## Description

La présente invention est relative à une composition de traitement cosmétique des cheveux, de type émulsion eau-dans-huile, comprenant dans un milieu cosmétiquement acceptable, au moins une huile non-volatile non-siliconée, au moins un tensioactif cationique, au moins un tensioactif non ionique différent des alcools gras en C₈-C₅₀ et au moins un alkylmonoglycoside ou alkylpolyglycoside, et à un procédé de traitement cosmétique des cheveux.

Les émulsions eau-dans-huile, notamment exemptes de composés siliconés, sont utilisées couramment en cosmétique et en particulier pour le soin de la peau car elles permettent de former un film lipidique à la surface de la peau la protégeant ainsi des agressions extérieures et prévenant la perte d'eau transépidermique.

Cependant, ces émulsions sont très peu utilisées dans le domaine capillaire, et en particulier celui du conditionnement des cheveux. En effet, elles présentent deux inconvénients majeurs, à savoir de ne pas pouvoir être rincées facilement et totalement, conduisant à un résidu gras inesthétique, et de ne pas permettre l'obtention d'un bon conditionnement du cheveu. En effet, on obtient généralement des cheveux ternes, collés et sales, et on observe un ramollissement de la fibre capillaire. Les cheveux sont en outre difficiles à démêler.

La demanderesse a découvert de manière surprenante que l'introduction d'une association particulière de tensioactifs dans une telle émulsion eau-dans-huile permettait non seulement d'améliorer sa rinçabilité et d'obtenir de bonnes propriétés cosmétiques, mais aussi d'obtenir une émulsion eau-dans-huile stable. Cette association particulière est constituée par au moins un tensioactif cationique, au moins un tensioactif non ionique différent des alcools gras en C₈-C₅₀ et de 0,01 à 10% en poids, par rapport au poids total de la composition, d'au moins un (alkyl en C₁₂₋₃₀) monoglycoside ou (alkyl en C₁₂₋₃₀)polyglycoside.

La présente invention a donc pour objet une composition de traitement des cheveux, de type émulsion eau-dans-huile, comprenant dans un milieu cosmétiquement acceptable, au moins une huile non-volatile non-siliconée, au moins un tensioactif cationique, au moins un tensioactif non ionique différent des alcools gras en C₈-C₅₀ et de 0,01 à 10% en poids, par rapport au poids total de la composition, d'au moins un (alkyl en C₁₂₋₃₀)monoglycoside ou (alkyl en C₁₂₋₃₀)polyglycoside.

Un autre objet de l'invention consiste en un procédé de traitement cosmétique des cheveux mettant en oeuvre une composition selon l'invention telle que décrite ci-dessous.

L'invention a encore pour objet une utilisation de la composition selon l'invention pour le conditionnement des cheveux et notamment comme après-shampoing.

D'autres objets, caractéristiques, aspects et avantages de l'invention apparaîtront encore plus clairement à la lecture de la description et des divers exemples qui suivent.

Selon l'invention, la composition de traitement cosmétique des cheveux, de type émulsion eau-dans-huile, comprend dans un milieu cosmétiquement acceptable, au moins une huile non-volatile non-siliconée, au moins un tensioactif cationique, au moins un tensioactif non ionique et de 0,01 à 10% en poids, par rapport au poids total de la composition, d'au moins un alkylmonoglycoside ou alkylpolyglycoside.

Par "milieu cosmétiquement acceptable", on entend un milieu compatible avec les cheveux.

Par "huile", on entend tout milieu non aqueux liquide à température ambiante (25°C±3°C) et sous pression atmosphérique, ayant une solubilité dans l'eau à 25°C inférieure à 0,5%.

Par "huile non-volatile", on entend une huile ayant une pression de vapeur à température ambiante (25°C±3°C) inférieure à 2,66 Pa (0,02mm de mercure).

Les huiles non-volatiles non-siliconées utilisables dans la présente invention sont notamment choisies parmi les huiles végétales, les huiles animales, les huiles minérales, les huiles synthétiques, les esters d'acide gras, et leurs mélanges.

Comme huile végétale, on peut notamment mentionner l'huile d'amande douce, l'huile d'avocat, l'huile de ricin, l'huile d'olive, la cire liquide de jojoba, l'huile de tournesol, l'huile de germes de blé, l'huile de sésame, l'huile d'arachide, l'huile de pépins de raisin, l'huile de soja, l'huile de colza, l'huile de carthame, l'huile de coprah, l'huile de maïs, l'huile de noisette, l'huile de palme, l'huile de noyau d'abricot et l'huile de calophyllum.

Comme huile animale, on peut notamment citer le perhydrosqualène.

La composition selon l'invention peut également comprendre une ou plusieurs huiles minérales telles qu'une huile de paraffine et l'huile de vaseline.

La composition selon l'invention peut également comprendre une ou plusieurs huiles synthétiques telles que le squalane, les poly(α-oléfines) comme l'isododécane ou l'isohexadécane, les huiles végétales transestérifiées et les huiles fluorées.

La composition selon l'invention peut également comprendre un ou plusieurs esters gras, tels que par exemple, les composés de formule RₐCOOR_{b} dans laquelle Rₐ représente le reste d'un acide supérieur linéaire ou ramifié, hydroxylé ou non, saturé ou non, comportant de 4 à 29 atomes de carbone et R_{b} représente une chaîne hydrocarbonée linéaire ou ramifiée, saturée ou non contenant de 3 à 30 atomes de carbone, le nombre total d'atomes de carbone de l'ester étant supérieur à 10. A titre d'exemples, on peut notamment citer l'huile de Purcellin (octanoate de stéaryle), le myristate d'isopropyle, le palmitate d'isopropyle, le stéarate de butyle, le laurate d'hexyle, l'isononanoate d'isononyle, le palmitate de 2-éthylhexyle, le laurate de 2-hexyldécyle, le palmitate de 2-octyldécyle, le myristate de 2-octyldodécyle, le néopentanoate d'isostéaryle ou le néopentanoate de tridécyle.

Les huiles particulièrement préférées dans la composition selon l'invention sont notamment choisies parmi l'huile d'avocat, l'isododécane, le myristate d'isopropyle et la cire liquide de jojoba.

L'huile ou les huiles telles que décrites ci-dessus sont notamment présentes dans la composition selon l'invention en une quantité allant de 0,1 à 30 % en poids, de préférence de 1 à 20 % en poids, et mieux encore de 5 à 15% en poids par rapport au poids total de la composition.

La composition selon l'invention comprend un ou plusieurs tensioactifs cationiques bien connus en soi, tels que les sels d'amines grasses primaires, secondaires ou tertiaires, éventuellement polyoxyalkylénées, les sels d'ammonium quaternaire, et leurs mélanges.

A titre de sels d'ammonium quaternaires, on peut notamment citer, par exemple :
- ceux répondant à la formule générale (I) suivante : 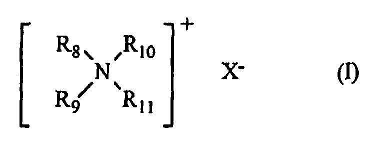
dans laquelle les radicaux R₈ à R₁₁, qui peuvent être identiques ou différents, représentent un radical aliphatique, linéaire ou ramifié, comportant de 1 à 30 atomes de carbone, ou un radical aromatique tel que aryle ou alkylaryle. Les radicaux aliphatiques peuvent comporter des hétéroatomes tels que notamment l'oxygène, l'azote, le soufre et les halogènes. Les radicaux aliphatiques sont par exemple choisis parmi les radicaux alkyle en C₁₋₃₀, alcoxy en C₁₋₃₀, polyoxyalkylène (C₂-C₆), alkylamide en C₁₋₃₀, alkyl(C₁₂-C₂₂)amidoalkyle(C₂-C₆), alkyl(C₁₂₋C₂₂)acétate, et hydroxyalkyle en C₁₋₃₀ ; X est un anion choisi dans le groupe des halogénures, phosphates, acétates, lactates, alkyl(C₂₋C₆)sulfates, alkyl- ou alkylaryl-sulfonates.

Parmi les sels d'ammonium quaternaire de formule (I), on préfère d'une part, les chlorures de tétraalkylammonium comme, par exemple, les chlorures de dialkyldiméthylammonium ou d'alkyltriméthylammonium dans lesquels le radical alkyle comporte environ de 12 à 22 atomes de carbone, en particulier les chlorures de béhényltriméthylammonium, de distéaryldiméthylammonium, de cétyltriméthylammonium, de benzyldiméthylstéarylammonium ou encore, d'autre part, le chlorure de palmitylamidopropyltriméthylammonium ou le chlorure de stéaramidopropyldiméthyl-(myristyl acétate)-ammonium commercialisé sous la dénomination CERAPHYL® 70 par la société VAN DYK.
- les sels d'ammonium quaternaire de l'imidazoline, comme par exemple ceux de formule (II) suivante : 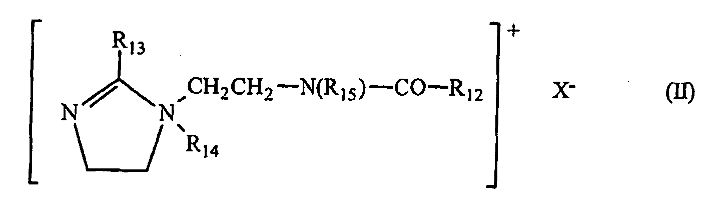
dans laquelle R₁₂ représente un radical alcényle ou alkyle comportant de 8 à 30 atomes de carbone, par exemple dérivés des acides gras du suif, R₁₃ représente un atome d'hydrogène, un radical alkyle en C₁-C₄ ou un radical alcényle ou alkyle comportant de 8 à 30 atomes de carbone, R₁₄ représente un radical alkyle en C₁-C₄, R₁₅ représente un atome d'hydrogène, un radical alkyle en C₁-C₄, X⁻ est un anion choisi dans le groupe des halogénures, phosphates, acétates, lactates, alkylsulfates, alkyl- ou alkylaryl-sulfonates. De préférence, R₁₂ et R₁₃ désignent un mélange de radicaux alcényle ou alkyle comportant de 12 à 21 atomes de carbone, par exemple dérivés des acides gras du suif, R₁₄ désigne un radical méthyle, R₁₅ désigne un atome d'hydrogène. Un tel produit est par exemple commercialisé sous la dénomination REWOQUAT® W 75 par la société REWO ;
- les sels de diammonium quaternaire de formule (III) : 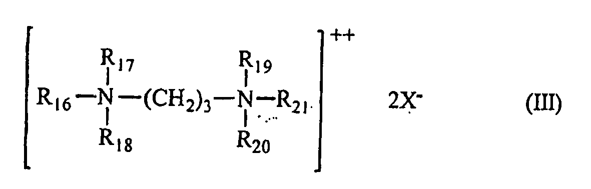
dans laquelle R₁₆ désigne un radical aliphatique comportant environ de 16 à 30 atomes de carbone ; R₁₇, R₁₈, R₁₉, R₂₀ et R₂₁, identiques ou différents sont choisis parmi un atome d'hydrogène et un radical alkyle comportant de 1 à 4 atomes de carbone ; et X est un anion choisi dans le groupe des halogénures, acétates, phosphates, nitrates et méthylsulfates. De tels sels de diammonium quaternaire comprennent notamment le dichlorure de propanesuif diammonium ;
- les sels d'ammonium quaternaire contenant au moins une fonction ester, tels que ceux de formule (IV) suivante : 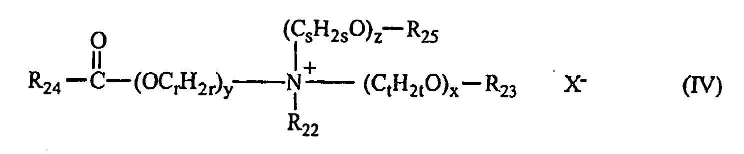
dans laquelle :
R₂₂ est choisi parmi les radicaux alkyles en C₁-C₆ et les radicaux hydroxyalkyles ou dihydroxyalkyles en C₁-C₆ ;
R₂₃ est choisi parmi :
   - le radical 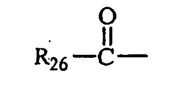
   - les radicaux R₂₇ hydrocarbonés en C₁-C₂₂, linéaires ou ramifiés, saturés ou insaturés,
   - l'atome d'hydrogène,
R₂₅ est choisi parmi :
   - le radical 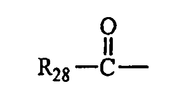
   - les radicaux R₂₉ hydrocarbonés en C₁-C₆, linéaires ou ramifiés, saturés ou insaturés,
   - l'atome d'hydrogène,
R₂₄, R₂₆ et R₂₈, identiques ou différents, sont choisis parmi les radicaux hydrocarbonés en C₇-C₂₁, linéaires ou ramifiés, saturés ou insaturés ;
r, s et t, identiques ou différents, sont des entiers valant de 2 à 6 ;
y est un entier valant de 1 à 10 ;
x et z, identiques ou différents, sont des entiers valant de 0 à 10 ;
X- est un anion simple ou complexe, organique ou inorganique ;
sous réserve que la somme x + y + z vaut de 1 à 15, que lorsque x vaut 0 alors R₂₃ désigne R₂₇ et que lorsque z vaut 0 alors R₂₅ désigne R₂₉.

Les radicaux alkyles R₂₂ peuvent être linéaires ou ramifiés et plus particulièrement linéaires.

De préférence R₂₂ désigne un radical méthyle, éthyle, hydroxyéthyle ou dihydroxypropyle, et plus particulièrement un radical méthyle ou éthyle.

Avantageusement, la somme x + y + z vaut de 1 à 10.

Lorsque R₂₃ est un radical R₂₇ hydrocarboné, il peut être long et avoir de 12 à 22 atomes de carbone, ou court et avoir de 1 à 3 atomes de carbone.

Lorsque R₂₅ est un radical R₂₉ hydrocarboné, il a de préférence 1 à 3 atomes de carbone.

Avantageusement, R₂₄, R₂₆ et R₂₈, identiques ou différents, sont choisis parmi les radicaux hydrocarbonés en C₁₁-C₂₁, linéaires ou ramifiés, saturés ou insaturés, et plus particulièrement parmi les radicaux alkyle et alcényle en C₁₁-C₂₁, linéaires ou ramifiés, saturés ou insaturés.

De préférence, x et z, identiques ou différents, valent 0 ou 1.

Avantageusement, y est égal à 1.

De préférence, r, s et t, identiques ou différents, valent 2 ou 3, et encore plus particulièrement sont égaux à 2.

L'anion est de préférence un halogénure (chlorure, bromure ou iodure) ou un alkylsulfate plus particulièrement méthylsulfate. On peut cependant utiliser le méthanesulfonate, le phosphate, le nitrate, le tosylate, un anion dérivé d'acide organique tel que l'acétate ou le lactate ou tout autre anion compatible avec l'ammonium à fonction ester.

L'anion X- est encore plus particulièrement le chlorure ou le méthylsulfate.

On utilise plus particulièrement dans la composition selon l'invention, les sels d'ammonium de formule (IV) dans laquelle :
- R₂₂ désigne un radical méthyle ou éthyle,
- x et y sont égaux à 1 ;
- z est égal à 0 ou 1 ;
- r, s et t sont égaux à 2 ;
- R₂₃ est choisi parmi :
   - le radical 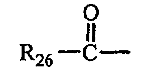
   - les radicaux méthyle, éthyle ou hydrocarbonés en C₁₄-C₂₂,
   - l'atome d'hydrogène ; 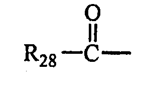
- R₂₅ est choisi parmi :
   - le radical
   - l'atome d'hydrogène ;
- R₂₄, R₂₆ et R₂₈, identiques ou différents, sont choisis parmi les radicaux hydrocarbonés en C₁₃-C₁₇, linéaires ou ramifiés, saturés ou insaturés, et de préférence parmi les radicaux alkyles et alcényles en C₁₃-C₁₇, linéaires ou ramifiés, saturés ou insaturés.

Avantageusement, les radicaux hydrocarbonés sont linéaires.

On peut citer par exemple les composés de formule (IV) tels que les sels (chlorure ou méthylsulfate notamment) de diacyloxyéthyl-diméthylammonium, de diacyloxyéthyl-hydroxyéthyl-méthylammonium, de monoacyloxyéthyl-dihydroxyéthyl-méthylammonium, de triacyloxyéthyl-méthylammonium, de monoacyloxyéthyl-hydroxyéthyl-diméthylammonium et leurs mélanges. Les radicaux acyles ont de préférence 14 à 18 atomes de carbone et proviennent plus particulièrement d'une huile végétale comme l'huile de palme ou de tournesol. Lorsque le composé contient plusieurs radicaux acyles, ces derniers peuvent être identiques ou différents.

Ces produits sont obtenus, par exemple, par estérification directe de la triéthanolamine, de la triisopropanolamine, d'alkyldiéthanolamine ou d'alkyldiisopropanolamine éventuellement oxyalkylénées sur des acides gras ou sur des mélanges d'acides gras d'origine végétale ou animale, ou par transestérification de leurs esters méthyliques. Cette estérification est suivie d'une quaternisation à l'aide d'un agent d'alkylation tel qu'un halogénure d'alkyle (méthyle ou éthyle de préférence), un sulfate de dialkyle (méthyle ou éthyle de préférence), le méthanesulfonate de méthyle, le para-toluènesulfonate de méthyle, la chlorhydrine du glycol ou du glycérol.

De tels composés sont par exemple commercialisés sous les dénominations DEHYQUART^{®} par la société HENKEL, STEPANQUAT^{®} par la société STEPAN, NOXAMIUM^{®} par la société CECA, REWOQUAT^{®} WE 18 par la société REWO-WITCO.

La composition selon l'invention contient de préférence un mélange de sels de mono-, di- et triester d'ammonium quaternaire avec une majorité en poids de sels de diester.

Comme mélange de sels d'ammonium, on peut utiliser par exemple le mélange contenant 15 à 30 % en poids de méthylsulfate d'acyloxyéthyl-dihydroxyéthyl-méthylammonium, 45 à 60% de méthylsulfate de diacyloxyéthyl-hydroxyéthyl-méthylammonium et 15 à 30% de méthylsulfate de triacyloxyéthyl-méthylammonium, les radicaux acyles ayant de 14 à 18 atomes de carbone et provenant d'huile de palme éventuellement partiellement hydrogénée.

On peut aussi utiliser les sels d'ammonium contenant au moins une fonction ester décrits dans les brevets US-A-4874554 et US-A-4137180.

Les tensioactifs cationiques particulièrement préférés dans la composition de l'invention sont choisis parmi les sels d'ammonium quaternaire, et en particulier parmi le chlorure de béhényltriméthylammonium et le chlorure de cétyltriméthylammonium.

La composition de traitement cosmétique des cheveux comprend de préférence le ou les tensioactifs cationiques en une quantité allant de 0,1 à 20 % en poids, mieux encore de 0,2 à 10 % en poids, et encore plus préférentiellement de 0,5 à 8 % en poids par rapport au poids total de la composition.

Les tensioactifs non-ioniques utilisables dans les compositions de la présente invention sont des composés bien connus en soi (voir notamment à cet égard "Handbook of Surfactants" par M.R. PORTER, éditions Blackie & Son (Glasgow and London), 1991, pp 116-178). Ils sont choisis notamment parmi les alcools polyéthoxylés, polypropoxylés ou polyglycérolés, les alpha-diols polyéthoxylés, polypropoxylés ou polyglycérolés, les alkyl(C₁₋₂₀)phénols polyéthoxylés, polypropoxylés ou polyglycérolés ou les acides gras polyéthoxylés, polypropoxylés ou polyglycérolés, la chaîne grasse comportant, par exemple, de 8 à 18 atomes de carbone, le nombre de groupements oxyde d'éthylène ou oxyde de propylène pouvant aller notamment de 2 à 50 et le nombre de groupements glycérol pouvant aller notamment de 2 à 30.

On peut également citer les condensats d'oxyde d'éthylène et d'oxyde de propylène sur des alcools gras ; les amides gras polyéthoxylés ayant de préférence de 2 à 30 motifs d'oxyde d'éthylène, les amides gras polyglycérolés comportant en moyenne de 1 à 5 groupements glycérol et en particulier de 1,5 à 4, les esters d'acides gras et de sorbitane éthoxylés ayant de 2 à 30 motifs d'oxyde d'éthylène, les esters d'acides gras du saccharose, les esters d'acides gras et de polyéthylèneglycol, les huiles végétales polyéthoxylées, les dérivés de N-(alkyl en C₆₋₂₄)glucamine, les oxydes d'amines tels que les oxydes d'(alkyl en C₁₀₋₁₄)amines ou les oxydes de N-(acyl en C₁₀₋₁₄)-aminopropylmorpholine.

Les tensioactifs non ioniques utilisés de préférence dans les compositions de l'invention présentent une HLB allant de 1,5 à 10, et mieux encore de 1,5 à 7.

La HLB ou balance hydrophile-lipophile du ou des tensioactifs non ioniques utilisés selon l'invention est la HLB selon GRIFFIN définie dans la publication J. Soc. Cosm. Chem. 1954 (Volume 5), pages 249-256.

A titres d'exemples de tensioactifs non ioniques présentant une HLB allant de 1,5 à 10, on peut notamment citer ceux vendus sous les marques commerciales suivantes :
Etocas 29 (HLB=1,7) par la société CRODA, Genapol PF 10 (HLB=2) par la société HOECHST, Synperonic PE L81 (HLB=2) par la société ICI, Prox-Onic EP 1090-1 (HLB=3) par la société PROTEX, Sinnopal DPN2 (HLB=3,3) par la société HENKEL, Antarox CA 210 (HLB=3,5) par la société RHONE-POULENC, Antarox O1P (HLB=3,5) par la société RHONE-POULENC, Alkasurf OP11 (HLB=3,6) par la société RHONE-POULENC, Triton X15 (HLB=3,6) par la société ROHM et HAAS, Alkasurf OP1 (HLB=3,6) par la société RHONE-POULENC, Arlacel 121 (HLB=3,8) par la société ICI, Prox-Onic HR ou HRH-05 (HLB=3,8) par la société PROTEX, Etocas 5 (HLB=3,9) par la société HOECHST, Genapol PF20 (HLB=4) par la société HOECHST, Imbentin N/7 A (HLB=4) par la société KOLB, Synperonic PE L122 (HLB=4) par la société ICI, Ethylan NP1 (HLB=4,5) par la société HARCROS, Imbentin N/020 (HLB=4,5) par la société KOLB, Kotilen O/3/020 (HLB=4,5) par la société KOLB, Synperonic PE L31 (HLB=4,5) par la société ICI, TO-55-A (HLB=4,5) par la société HEFTI, Alkasurf NP-1 (HLB=4,6) par la société RHONE-POULENC, Antarox CO 210 (HLB=4,6) par la société RHONE-POULENC, Prox-Onic NP-1 (HLB=4,6) par la société PROTEX, Rhodiasurf NP2 (HLB=4,6) par la société RHONE-POULENC, Soprophor BC2 (HLB=4,6) par la société RHONE-POULENC, Triton N17 (HLB=4,6) par la société ROHM et HAAS, Akyporox NP15 (HLB=4,7) par la société CHEM-Y, Texofor M2 (HLB=4,8) par la société RHONE-POULENC, Alkasurf SA2 (HLB=4,9) par la société RHONE-POULENC, Arlacel 989 (HLB=4,9) par la société ICI, Brij 72 (HLB=4,9) par la société ICI, Brij 92 (HLB=4,9) par la société ICI, Brij 93 (HLB=4,9) par la société ICI, Prox-Onic SA-1 ou 2/02 (HLB=4,9) par la société PROTEX, Simulsol 72 (HLB=4,9) par la société SEPPIC, Simulsol 92 (HLB=4,9) par la société SEPPIC, Volpo S-2 (HLB=4,9) par la société CRODA, Arlacel 581 (HLB=5,0) par la société ICI, Arlacel 582 (HLB=5,0) par la société ICI, Genapol 0-020 (HLB=5,0) par la société HOECHST, Imbentin POA/020 (HLB=5,0) par la société KOLB, et Mergital Q2 (HLB=5,0) par la société HENKEL, Imbentin POA/024 (HLB=5,5) par la société ICI, Synperonic PE L92 (HLB= 5,5) par la société ICI, Mergital LM2 (HLB=5,8) par la société HENKEL, Atlas G-70140 (HLB=6) par la société ICI, Imbentin. AG/124S/ 020 (HLB=6) par la société KOLB, Imbentin. L/125/025 HLB=6 par la société KOLB, Simulsol 989 (HLB=6) par la société SEPPIC, Soprophor HR10 (HLB=6) par la société RHONE POULENC, Kotilen O/1/050 (HLB=6,2)par la société KOLB, Croduret 10 (HLB=6,3) par la société CRODA, Etocas 10 (HLB=6,3) par la société CRODA, Imbentin OA/030 (HLB=6,3) par la société KOLB, Soprophor 208 (HLB=6,9) par la société RHONE POULENC, Ethylan 172 (HLB=7) par la société HARCROS, Akyporox NP 40 (HLB=7,1) par la société CHEM-Y, Polychol 5 (HLB=7,3) par la société CRODA, Arlatone 985 (HLB=7,5) par la société ICI, Sandoxylate FOL4 (HLB=7,5) par la société SANDOZ, Radiasurf 7453 (HLB=7,8) par la société OLEOFINA, Prox-onic OA-1/04 (HLB=7,9) par la société PROTEX, Prox-onic TD-1/03 (HLB=7,9) par la société PROTEX, Genapol PF 40 (HLB=8) par la société HOECHST, PGE-400 - DS (HLB=8) par la société HEFTI, PGE-400- DO (HLB=8) par la société HEFTI, Sapogenat 6-040 (HLB=8) par la société HOECHST, Intrasol FA28/50/4 (HLB=8,1) par la société STOCKHAUSEN, Serdox NOG 200 S (HLB=8,5) par la société SERVO, Berol 26 (HLB=8,9) par la société BEROL NOBEL, Genapol O-050 (HLB=9) par la société HOECHST, Prox-onic LA-1/04 (HLB=9,2) par la société PROTEX, Eumulgin 05 (HLB=9,5) par la société HENKEL, Etocas 20 (HLB=9,6) par la société CRODA, Antarox CO 520 (HLB=10) par la société RHONE POULENC, Imbentin POA/060 (HLB=10) par la société KOLB, TO-55-EL (HLB=10) par la société HEFTI.

Les tensioactifs non ioniques plus particulièrement préférés sont choisis parmi l'isostéarate de sorbitane, l'isostéarate de polyglycéryle, le dioléate de méthylglucose, l'huile de ricin hydrogénée polyéthoxylée avec 7 moles d'oxyde d'éthylène et leurs mélanges.

Le ou les tensioactifs non ioniques tels que définis ci-dessus sont contenus de préférence en une quantité allant de 0,01 à 10 % en poids, de préférence de 0,1 à 5 % en poids, et mieux encore de 0,2 à 3 % en poids par rapport au poids total de la composition.

Les alkylmonoglycosides ou alkylpolyglycosides particulièrement préférés dans l'invention sont ceux dont le groupe alkyle comporte de 16 à 24 atomes de carbone.

A titre d'exemples particulièrement préférés, on peut notamment citer l'arachidylglycoside

Le ou les (alkyl en C₁₂₋₃₀)monoglycosides ou (alkyl en C₁₂₋₃₀)polyglycosides sont contenus en une quantité allant de 0,01 à 10 % en poids, de préférence de 0,02 à 5 % en poids, et mieux encore de 0,05 à 1 % en poids par rapport au poids total de la composition.

De préférence, le rapport pondéral huile(s)/tensioactif(s) non ionique(s) des compositions de l'invention est compris entre 3 et 100, mieux encore entre 10 et 75 et encore plus préférentiellement entre 15 et 40.

Le milieu aqueux cosmétiquement acceptable comprend l'eau ou un mélange d'eau et d'un solvant cosmétiquement acceptable choisi parmi les alcools inférieurs en C₁-C₄, tels que l'éthanol, l'isopropanol, le tertio-butanol ou le n-butanol ; les polyols comme le propylèneglycol ; les éthers de polyols ; les alcanes en C₅-C₁₀ ; les cétones en C₃₋₄ comme l'acétone et la méthyléthylcétone ; les acétates d'alkyle en C₁-C₄ comme l'acétate de méthyle, l'acétate d'éthyle et l'acétate de butyle ; le diméthoxyéthane, le diéthoxyéthane ; et leurs mélanges.

Les compositions selon l'invention peuvent comprendre en outre au moins un alcool gras en C₁₄₋₃₀, et de préférence au moins l'un des alcools myristylique, cétylique, stéarylique, arachidylique, béhénylique et érucylique. Ils sont généralement présents en une quantité inférieure à 10 % en poids, de préférence allant de 0,01 à 5 % en poids, et mieux encore de 0,05 à 1,5 % en poids par rapport au poids total de la composition.

Les compositions selon l'invention peuvent également comprendre en outre au moins une huile siliconée bien connue dans la technique, en une quantité inférieure à 10 % en poids, de préférence allant de 0,01 à 8%, et encore plus préférentiellement de 0,1 à 5% en poids par rapport au poids total de la composition.

A titre d'exemples d'huile siliconée, on peut notamment citer les polydiméthylsiloxanes linéaires ou cycliques.

Les compositions selon l'invention peuvent également contenir au moins un additif tel qu'un polymère cationique, anionique, non ionique ou amphotère ; un épaississant polymérique naturel ou synthétique, anionique, amphotère, zwittérionique, non ionique ou cationique, associatif ou non ; un épaississant non polymérique comme un électrolyte ou un sucre ; un nacrant ; un opacifiant ; un filtre solaire ; un parfum ; un colorant ; une particule organique ou minérale ; un conservateur ; ou un agent de stabilisation du pH.

L'homme de métier veillera à choisir les éventuels additifs et leur quantité de manière à ce qu'ils ne nuisent pas aux propriétés des compositions de la présente invention.

Ces additifs sont présents dans la composition selon l'invention en une quantité allant de 0 à 50 % en poids par rapport au poids total de la composition.

De préférence, la composition selon l'invention contient moins de 5% en poids de tensioactif anionique, et encore plus préférentiellement moins de 1 % en poids. Selon un mode de réalisation particulièrement avantageux, la composition selon l'invention ne contient pas de tensioactif anionique.

La taille moyenne des particules de l'émulsion est avantageusement comprise entre 300 nanomètres et 50 micromètres, de préférence entre 500 nanomètres et 20 micromètres, et encore plus préférentiellement entre 750 nanomètres et 10 micromètres.

Les compositions selon l'invention peuvent se présenter sous forme de liquides fluides ou épaissis, de gels, de crèmes, ou d'émulsions simples ou multiples.

Les compositions peuvent être utilisées, par exemple, dans des shampoings, produits de coloration ou de décoloration ou de permanente, produits de coiffage, soins rincés, masques de soin profond, gels douches, lotions ou crèmes de traitement du cuir chevelu, ou encore déposées sur des lingettes.

La présente invention concerne également un procédé de traitement cosmétique des cheveux qui consiste à appliquer une quantité efficace d'une composition telle que décrite ci-dessus, sur les cheveux, à effectuer un éventuel rinçage après un éventuel temps de pose.

Selon un mode de réalisation préféré de l'invention, la composition peut être utilisée pour le conditionnement des cheveux et plus particulièrement comme après-shampoing.

Les exemples suivants sont donnés à titre illustratif de la présente invention.

### EXEMPLES

On a préparé les émulsions eau-dans-huile en mélangeant les ingrédients indiqués dans le tableau ci-dessous dans les proportions indiquées en % en poids par rapport au poids total de l'émulsion.

| | Ex. 1 | Ex. 2 | Ex. 3 |
|---|---|---|---|
| Myristate d'isopropyle | 9,25 | 9,3 | 8 |
| Isoparaffine en C₁₁₋₁₂ | - | - | 1,5 |
| Huile d'avocat | - | - | - |
| Isostéarate de 3-polyglycéryle et isostéarate de sorbitane⁽¹⁾ | 0,75 | - | - |
| Dioléate de méthylglucose⁽²⁾ | - | 0,7 | - |
| Huile de ricin hydrogénée de polyéthylèneglycol⁽³⁾ | - | - | 0,85 |
| Arachidylglucoside (à 15 % de MA)⁽⁴⁾ | 0,15 MA | 0,15 MA | 0,1 MA |
| Chlorure de béhényltriméthyl-ammonium (à 80 % de MA) ⁽⁵⁾ | 4 MA | 3 MA | 4 MA |
| Propylène glycol | - | 2 | 2 |
| Eau qsp | 100 | 100 | 100 |

| | | | |
|---|---|---|---|
| MA : Matière Active ⁽¹⁾ : vendu sous le nom commercial Arlacel 1690 par UNIQUEMA ⁽²⁾ : vendu sous le nom commercial Glucate DO par AMERCHOL ⁽³⁾ : vendu sous le nom commercial Arlacel 989 par UNQUEMA ⁽⁴⁾ : vendu sous le nom commercial Montanov 202 par SEPPIC ⁽⁵⁾ : vendu sous le nom commercial Genamin KDMP par CLARIANT | | | |

On a appliqué les différentes émulsions sur des cheveux sensibilisés.

On observe alors une bonne rinçabilité et un bon conditionnement de la fibre.

## Revendications

1. Composition de traitement cosmétique des cheveux, de type émulsion eau-dans-huile, **caractérisée en ce qu'**elle comprend dans un milieu cosmétiquement acceptable, au moins une huile non-volatile non-siliconée, au moins un tensioactif cationique, au moins un tensioactif non-ionique différent des alcools gras en C₈-C₅₀, et de 0,01 à 10% en poids, par rapport au poids total de la composition, d'au moins un (alkyl en C₁₂₋₃₀)monoglycoside ou (alkyl en C₁₂₋₃₀)polyglycoside.

2. Composition de traitement cosmétique des cheveux selon la revendication 1, **caractérisée en ce que** l'huile non-volatile non-siliconée est une huile végétale, une huile animale, une huile minérale, une huile synthétique, un ester d'acide gras ou un de leurs mélanges.

3. Composition de traitement cosmétique des cheveux selon la revendication 2, **caractérisée en ce que** l'huile végétale est choisie parmi l'huile d'amande douce, l'huile d'avocat, l'huile de ricin, l'huile d'olive, la cire liquide de jojoba, l'huile de tournesol, l'huile de germes de blé, l'huile de sésame, l'huile d'arachide, l'huile de pépins de raisin, l'huile de soja, l'huile de colza, l'huile de carthame, l'huile de coprah, l'huile de maïs, l'huile de noisette, l'huile de palme, l'huile de noyau d'abricot et l'huile de calophyllum.

4. Composition de traitement cosmétique des cheveux selon la revendication 2, **caractérisée en ce que** l'huile animale est le perhydrosqualène.

5. Composition de traitement cosmétique des cheveux selon la revendication 2, **caractérisée en ce que** l'huile minérale est choisie parmi l'huile de paraffine et l'huile de vaseline.

6. Composition de traitement cosmétique des cheveux selon la revendication 2, **caractérisée en ce que** l'huile synthétique est choisie parmi le squalane, les poly(α-oléfines), les huiles végétales transestérifiées et les huiles fluorées.

7. Composition de traitement cosmétique des cheveux selon la revendication 2, **caractérisée en ce que** l'ester gras est choisi parmi l'huile de Purcellin, le myristate d'isopropyle, le palmitate d'isopropyle, le stéarate de butyle, le laurate d'hexyle, l'isononanoate d'isononyle, le palmitate de 2-éthylhexyle, le laurate de 2-hexyldécyle, le palmitate de 2-octyldécyle, le myristate de 2-octyldodécyle, le néopentanoate d'isostéaryle ou le néopentanoate de tridécyle.

8. Composition de traitement cosmétique des cheveux selon la revendication 1 ou 2, **caractérisée en ce que** l'huile non-volatile non-siliconée est choisie parmi l'huile d'avocat, l'isododécane, le myristate d'isopropyle et la cire liquide de jojoba.

9. Composition de traitement cosmétique des cheveux selon l'une quelconque des revendications précédentes, **caractérisée en ce qu'**elle comprend l'huile ou les huiles en une quantité allant de 0,1 à 30 % en poids, de préférence de 1 à 20% en poids, et mieux encore de 5 à 15% en poids par rapport au poids total de la composition.

10. Composition de traitement cosmétique des cheveux selon l'une quelconque des revendications précédentes, **caractérisée en ce que** les tensioactifs cationiques sont choisis parmi les sels d'amines grasses primaires, secondaires ou tertiaires, éventuellement polyoxyalkylénées, les sels d'ammonium quaternaire et leurs mélanges.

11. Composition de traitement cosmétique des cheveux selon la revendication 10, **caractérisée en ce que** les sels d'ammonium quaternaire sont choisis parmi :
- ceux répondant à la formule générale (I) suivante : 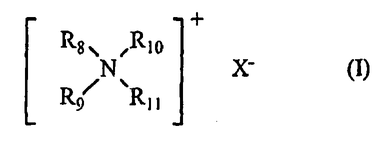 dans laquelle les radicaux R₈ à R₁₁, qui peuvent être identiques ou différents, représentent un radical aliphatique, linéaire ou ramifié, comportant de 1 à 30 atomes de carbone, ou un radical aromatique ; X est un anion choisi dans le groupe des halogénures, phosphates, acétates, lactates, alkyl(C₂-C₆)sulfates, alkyl- ou alkylaryl-sulfonates ;
- les sels d'ammonium quaternaire de l'imidazoline ;
- les sels de diammonium quaternaire de formule (III) : 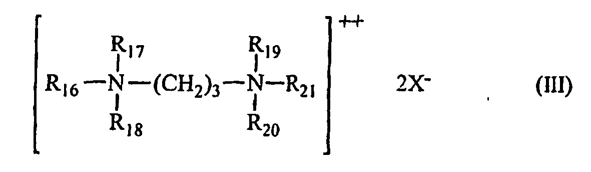 dans laquelle R₁₆ désigne un radical aliphatique comportant environ de 16 à 30 atomes de carbone ; R₁₇, R₁₈, R₁₉, R₂₀ et R₂₁, identiques ou différents sont choisis parmi un atome d'hydrogène et un radical alkyle comportant de 1 à 4 atomes de carbone ; et X est un anion choisi dans le groupe des halogénures, acétates, phosphates, nitrates et méthylsulfates ;
- les sels d'ammonium quaternaire contenant au moins une fonction ester.

12. Composition de traitement cosmétique des cheveux selon la revendication 10 ou 11, **caractérisée en ce que** le tensioactif cationique est choisi parmi les chlorures de behenyltriméthylammonium et de cétyltriméthylammonium.

13. Composition de traitement cosmétique des cheveux selon l'une quelconque des revendications précédentes, **caractérisée en ce qu'**elle comprend le(s) tensioactif(s) cationique(s) en une quantité allant de 0,1 à 20 % en poids, de préférence de 0,2 à 10 % et encore plus préférentiellement de 0,5 à 8% en poids par rapport au poids total de la composition.

14. Composition de traitement cosmétique des cheveux selon l'une quelconque des revendications précédentes, **caractérisée en ce que** le tensioactif non ionique présente un HLB allant de 1,5 à 10.

15. Composition de traitement cosmétique des cheveux selon la revendication 14, **caractérisée en ce que** le HLB va de 1,5 à 7.

16. Composition de traitement selon l'une quelconque des revendications précédentes, **caractérisée en ce que** le tensioactif non ionique est choisi parmi les alcools polyéthoxylés, polypropoxylés ou polyglycérolés ; les alpha-diols polyéthoxylés, polypropoxylés ou polyglycérolés ; les alkyl(C₁₋₂₀)phénols polyéthoxylés, polypropoxylés ou polyglycérolés ou les acides gras polyéthoxylés, polypropoxylés ou polyglycérolés ; les condensats d'oxyde d'éthylène et d'oxyde de propylène sur des alcools gras ; les amides gras polyéthoxylés ; les amides gras polyglycérolés comportant en moyenne de 1 à 5 groupements glycérol ; les esters d'acides gras et de sorbitane éthoxylés ayant de 2 à 30 motifs d'oxyde d'éthylène ; les esters d'acides gras du saccharose ; les esters d'acides gras et de polyéthylèneglycol ; les huiles végétales polyéthoxylées ; les dérivés de N-(alkyl en C₆₋₂₄)glucamine ; et les oxydes d'amines.

17. Composition de traitement cosmétique des cheveux selon la revendication 16, **caractérisée en ce que** le tensioactif non ionique est choisi parmi l'isostéarate de sorbitane, l'isostéarate de polyglycéryle, le dioléate de méthylglucose et l'huile de ricin hydrogénée polyéthoxylée avec 7 moles d'oxyde d'éthylène.

18. Composition de traitement cosmétique des cheveux selon l'une quelconque des revendications précédentes, **caractérisée en ce qu'**elle comprend le(s) tensioactif(s) non ionique(s) en une quantité allant de 0,01 à 10 % en poids, de préférence de 0,1 à 5 % en poids par rapport au poids total de la composition.

19. Composition de traitement cosmétique des cheveux selon l'une quelconque des revendications précédentes, **caractérisée en ce que** l'(alkyl en C₁₂₋₃₀)monoglycoside ou (alkyl en C₁₂₋₃₀)polyglycoside est choisi parmi ceux dont le groupe alkyle comporte de 16 à 24 atomes de carbone.

20. Composition de traitement cosmétique des cheveux selon l'une quelconque des revendications précédentes, **caractérisée en ce qu'**elle comprend le(s) (alkyl en C₁₂₋₃₀)monoglycoside(s) ou (alkyl en C₁₂₋₃₀)polyglycoside(s) en une quantité allant de 0,02 à 5 % en poids par rapport au poids total de la composition.

21. Composition de traitement cosmétique des cheveux selon l'une quelconque des revendications précédentes, **caractérisée en ce que** le rapport pondéral huile(s)/tensioactif(s) non ionique(s) est compris entre 3 et 100, de préférence entre 10 et 75.

22. Composition de traitement cosmétique des cheveux selon l'une quelconque des revendications précédentes, **caractérisée en ce qu'**elle comprend en outre au moins un alcool gras en C₁₄₋₃₀.

23. Composition de traitement cosmétique des cheveux selon la revendication 22, **caractérisée en ce que** l'alcool gras en C₁₄₋₃₀ est choisi parmi les alcools myristylique, cétylique, stéarylique, arachidylique, béhénylique et érucylique.

24. Composition de traitement cosmétique des cheveux selon la revendication 22 ou 23, **caractérisée en ce qu'**elle comprend le(s) alcool(s) gras en une quantité inférieure à 10 % en poids par rapport au poids total de la composition.

25. Composition de traitement cosmétique des cheveux selon l'une quelconque des revendications précédentes, **caractérisée en ce que** le milieu cosmétiquement acceptable comprend l'eau ou un mélange d'eau et d'au moins un solvant cosmétiquement acceptable.

26. Composition de traitement cosmétique des cheveux selon la revendication 25, **caractérisée en ce que** le solvant cosmétiquement acceptable est choisi parmi les alcools inférieurs en C₁-C₄, les polyols, les éthers de polyols, les alcanes en C₅-C₁₀, les cétones en C₃₋₄, les acétates d'alkyle en C₁-C₄, le diméthoxyéthane et le diéthoxyéthane.

27. Composition de traitement cosmétique des cheveux selon l'une quelconque des revendications précédentes, **caractérisée en ce qu'**elle comprend en outre au moins une huile siliconée en une quantité inférieure à 10 % en poids par rapport au poids total de la composition.

28. Composition de traitement cosmétique des cheveux selon l'une quelconque des revendications précédentes, **caractérisée en ce qu'**elle comprend au moins un additif choisi parmi les polymères cationiques, anioniques, non ioniques ou amphotères ; les épaississants polymériques naturels ou synthétiques, anioniques, amphotères, zwittérioniques, non ioniques ou cationiques, associatifs ou non ; les épaississants non polymériques ; les nacrants ; les opacifiants ; les filtres solaires ; les parfums ; les colorants ; les particules organiques ou minérales ; les conservateurs ; et les agents de stabilisation du pH.

29. Procédé de traitement cosmétique des cheveux, **caractérisé en ce que** l'on applique sur les cheveux une composition de traitement cosmétique selon l'une quelconque des revendications précédentes.

30. Utilisation d'une composition de traitement cosmétique selon l'une quelconque des revendications 1 à 28, pour le conditionnement des cheveux.

31. Utilisation d'une composition de traitement cosmétique selon la revendication 30, comme après-shampoing.

## Patentansprüche

1. Zusammensetzung zur kosmetischen Behandlung der Haare vom Typ einer Wasser-in-Öl-Emulsion, **dadurch gekennzeichnet, dass** sie in einem kosmetischen akzeptablen Medium enthält:
mindestens ein nichtflüchtiges Öl ohne Silicongruppen,
mindestens ein kationisches Tensid, mindestens ein nichtionisches Tensid und in einem Mengenanteil von 0,01 bis 10 Gew.-%, bezogen auf das Gesamtgewicht der Zusammensetzung, mindestens ein (C₁₂₋₃₀-Alkyl)monoglycosid oder (C₁₂₋₃₀-Alkyl)polyglycosid.

2. Zusammensetzung zur kosmetischen Behandlung der Haare nach Anspruch 1, **dadurch gekennzeichnet, dass** das nichtflüchtige Öl ohne Silicongruppen ein Pflanzenöl, ein tierisches Öl, ein Mineralöl, ein synthetisches Öl oder ein Fettsäureester oder ein Gemisch dieser Verbindungen ist.

3. Zusammensetzung zur kosmetischen Behandlung der Haare nach Anspruch 2, **dadurch gekennzeichnet, dass** das Pflanzenöl ausgewählt ist unter Süßmandelöl, Avocadoöl, Ricinusöl, Olivenöl, flüssigem Jojobawachs, Sonnenblumenöl, Weizenkeimöl, Sesamöl, Erdnussöl, Traubenkernöl, Sojaöl, Rapsöl, Distelöl, Coprahöl, Maisöl, Haselnussöl, Palmöl, Aprikosenkernöl und Calophyllumöl.

4. Zusammensetzung zur kosmetischen Behandlung der Haare nach Anspruch 2, **dadurch gekennzeichnet, dass** das tierische Öl Perhydrosqualen ist.

5. Zusammensetzung zur kosmetischen Behandlung der Haare nach Anspruch 2, **dadurch gekennzeichnet, dass** das Mineralöl unter Paraffinöl und Vaselinöl ausgewählt ist.

6. Zusammensetzung zur kosmetischen Behandlung der Haare nach Anspruch 2, **dadurch gekennzeichnet, dass** das synthetische Öl unter Squalan, Poly(α-olefinen), umgeesterten Pflanzenölen und fluorierten Ölen ausgewählt ist.

7. Zusammensetzung zur kosmetischen Behandlung der Haare nach Anspruch 2, **dadurch gekennzeichnet, dass** der Fettsäureester ausgewählt ist unter Purcellinöl, Isopropylmyristat, Isopropylpalmitat, Butylstearat, Hexyllaurat, Isononylisononanoat, 2-Ethylhexylpalmitat, 2-Hexyldecyllaurat, 2-Octyldecylpalmitat, 2-Octyldodecylmyristat, Isostearylneopentanoat oder Tridecylneopentanoat.

8. Zusammensetzung zur kosmetischen Zusammensetzung der Haare nach Anspruch 1 oder 2, **dadurch gekennzeichnet, dass** das nichtflüchtige Öl ohne Silicongruppen ausgewählt ist unter Avocadoöl, Isododecan, Isopropylmyristat und flüssigem Jojobawachs.

9. Zusammensetzung zur kosmetischen Behandlung der Haare nach einem der vorhergehenden Ansprüche, **dadurch gekennzeichnet, dass** sie das Öl oder die Öle in einem Mengenanteil von 0,1 bis 30 Gew.-%, bevorzugt von 1 bis 20 Gew.-% und noch günstiger von 5 bis 15 Gew.-% enthält, bezogen auf das Gesamtgewicht der Zusammensetzung.

10. Zusammensetzung zur kosmetischen Behandlung der Haare nach einem der vorhergehenden Ansprüche, **dadurch gekennzeichnet, dass** die kationischen Tenside unter Salzen von primären, sekundären oder tertiären und gegebenenfalls polyoxyalkylenierten Fettaminen und quaternären Ammoniumsalzen sowie Gemischen dieser Verbindungen ausgewählt sind.

11. Zusammensetzung zur kosmetischen Behandlung der Haare nach Anspruch 10, **dadurch gekennzeichnet, dass** die quaternären Ammoniumsalze ausgewählt sind unter
- quaternären Ammoniumsalzen der nachstehenden allgemeinen Formel (I): 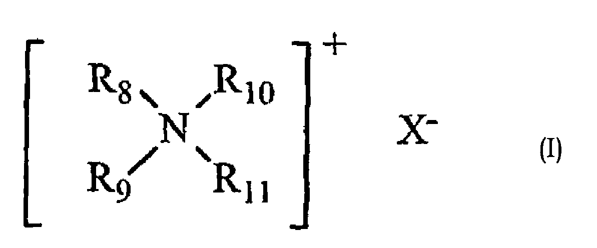 in der bedeuten:
R₈ bis R₁₁, die gleich oder verschieden sein können, eine geradkettige oder verzweigte aliphatische Gruppe mit 1 bis 30 Kohlenstoffatomen oder eine aromatische Gruppe, X ein Anion, das aus der Gruppe der Hologenide, Phosphate, Acetate, Lactate, C₂-C₆-Alkylsulfate,
Alkylsulfonate oder Alkylarylsulfonate ausgewählt ist;
- quaternären Ammoniumsalzen des Imidazolins;
- quaternären Diammoniumsalzen der Formel (III): 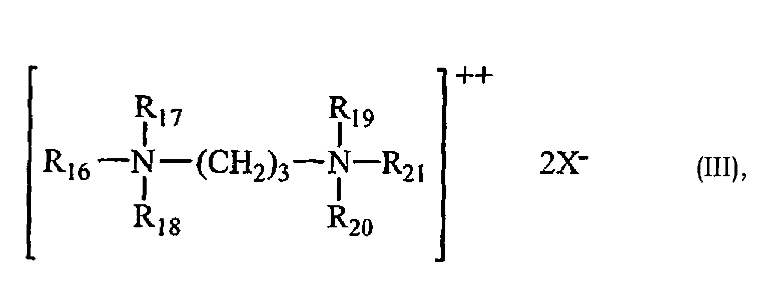 in der bedeuten:
R₁₆ eine aliphatische Gruppe mit etwa 16 bis 30 Kohlenstoffatomen;
R₁₇, R₁₈, R₁₉, R₂₀ und R₂₁, die gleich oder verschieden sind, ein Wasserstoffatom oder eine Alkylgruppe mit 1 bis 4 Kohlenstoffatomen,
X ein Anion, das aus der Gruppe der Halogenide, Acetate, Phosphate, Nitrate und Methylsulfate ausgewählt ist;
- quaternären Ammoniumsalzen, die mindestens eine Esterfunktion enthalten.

12. Zusammensetzung zur kosmetischen Behandlung der Haare nach Anspruch 10 oder 11, **dadurch gekennzeichnet, dass** das kationische Tensid unter Behenyltrimethylammoniumchlorid und Cetyltrimethylammoniumchlorid ausgewählt ist.

13. Zusammensetzung zur kosmetischen Behandlung der Haare nach einem der vorhergehenden Ansprüche, **dadurch gekennzeichnet, dass** sie das kationische Tensid oder die kationischen Tenside in einem Mengenanteil von 0,1 bis 20 Gew.-%, bevorzugt von 0,2 bis 10 Gew.-% und noch bevorzugter von 0,5 bis 8 Gew.-% enthält, bezogen auf das Gesamtgewicht der Zusammensetzung.

14. Zusammensetzung zur kosmetischen Behandlung der Haare nach einem der vorhergehenden Ansprüche, **dadurch gekennzeichnet, dass** das nichtionische Tensid einen HLB-Wert von 1,5 bis 10 aufweist.

15. Zusammensetzung zur kosmetischen Behandlung der Haare nach Anspruch 14, **dadurch gekennzeichnet, dass** der HLB-Wert im Bereich von 1,5 bis 7 liegt.

16. Zusammensetzung zur Behandlung nach einem der vorhergehenden Ansprüche, **dadurch gekennzeichnet, dass** das nichtionische Tensid ausgewählt ist unter polyethoxylierten, polypropoxylierten oder polyglycerylierten Alkoholen; polyethoxylierten, polypropoxylierten oder polyglycerylierten α-Diolen, polyethoxylierten, polypropoxylierten oder polyglycerylierten C₁₋₂₀-Alkylphenolen oder polyethoxylierten, polypropoxylierten oder polyglycerylierten Fettsäuren; Kondensationsprodukten von Ethylenoxid und Propylenoxid mit Fettalkoholen; polyethoxylierten Fettamiden; polyglycerylierten Fettamiden, die im Mittel 1 bis 5 Glycerylgruppen enthalten; ethoxylierten Sorbitan-Fettsäureestern mit 2 bis 30 Ethylenoxideinheiten; Fettsäureestern von Saccharose; Fettsäureestern von Polyethylenglycol; polyethoxylierten Pflanzenölen; N-(C₆₋₂₄-Alkyl)-glucamin-Derivaten und Aminoxiden.

17. Zusammensetzung zur kosmetischen Behandlung der Haare nach Anspruch 16, **dadurch gekennzeichnet, dass** das nichtionische Tensid unter Sorbitanisostearat, Polyglycerylisostearat, Methylglucosedioleat und polyethoxyliertem hydriertem Ricinusöl mit 7 mol Ethylenoxid ausgewählt ist.

18. Zusammensetzung zur kosmetischen Behandlung der Haare nach einem der vorgehenden Ansprüche, **dadurch gekennzeichnet, dass** sie das nichtionische Tensid oder die nichtionischen Tenside in einem Mengenanteil von 0,01 bis 10 Gew.-% und bevorzugt von 0,1 bis 5 Gew.-% enthält, bezogen auf das Gesamtgewicht der Zusammensetzung.

19. Zusammensetzung zur kosmetischen Behandlung der Haare nach einem der vorhergehenden Ansprüche, **dadurch gekennzeichnet, dass** das C₁₂₋₃₀-Alkylmonoglycosid oder das C₁₂₋₃₀-Alkylpolyglycosid unter entsprechenden Verbindungen ausgewählt ist, deren Alkylgruppe 16 bis 24 Kohlenstoffatome aufweist.

20. Kosmetische Zusammensetzung zur Behandlung der Haare nach einem der vorhergehenden Ansprüche, **dadurch gekennzeichnet, dass** sie das (C₁₂₋₃₀-Alkyl)monoglycosid oder die (C₁₂₋₃₀-Alkyl)monoglycoside oder das (C₁₂₋₃₀-Alkyl)polyglycosid oder die (C₁₂₋₃₀-Alkyl)polyglycoside in einem Mengenanteil von 0,02 bis 5 Gew.-% enthält, bezogen auf das Gesamtgewicht der Zusammensetzung.

21. Zusammensetzung zur kosmetischen Behandlung der Haare nach einem der vorhergehenden Ansprüche, **dadurch gekennzeichnet, dass** das Gewichtsverhältnis Öl(e)/nichtionische(s) Tensid(e) im Bereich von 3 bis 100 und bevorzugt im Bereich von 10 bis 75 liegt.

22. Zusammensetzung zur kosmetischen Behandlung der Haare nach einem der vorhergehenden Ansprüche, **dadurch gekennzeichnet, dass** sie ferner mindestens einen C₁₄₋₃₀-Fettalkohol enthält.

23. Zusammensetzung zur kosmetischen Behandlung der Haare nach Anspruch 22, **dadurch gekennzeichnet, dass** der C₁₄₋₃₀-Fettalkohol unter Myristylalkohol, Cetylalkohol, Stearylalkohol, Arachidylalkohol, Behenylalkohol und Erucylalkohol ausgewählt ist.

24. Zusammensetzung zur kosmetischen Behandlung der Haare nach Anspruch 22 oder 23, **dadurch gekennzeichnet, dass** sie den Fettalkohol oder die Fettalkohole in einer Menge von weniger als 10 Gew.-% enthält, bezogen auf das Gesamtgewicht der Zusammensetzung.

25. Zusammensetzung zur kosmetischen Behandlung der Haare nach einem der vorhergehenden Ansprüche, **dadurch gekennzeichnet, dass** das kosmetisch akzeptable Medium Wasser oder ein Gemisch von Wasser mit mindestens einem kosmetisch akzeptablen Lösungsmittel umfasst.

26. Zusammensetzung zur kosmetischen Behandlung der Haare nach Anspruch 25, **dadurch gekennzeichnet, dass** das kosmetisch akzeptable Lösungsmittel unter niederen C₁-C₄-Alkoholen, Polyolen, Polyolethern, C₅-C₁₀-Alkanen, C₃₋₄-Ketonen, C₁-C₄-Alkylacetaten, Dimethoxyethan und Diethoxyethan ausgewählt ist.

27. Zusammensetzung zur kosmetischen Behandlung der Haare nach einem der vorhergehenden Ansprüche, **dadurch gekennzeichnet, dass** sie ferner mindestens ein Siliconöl in einem Mengenanteil von weniger als 10 Gew.-% enthält, bezogen auf das Gesamtgewicht der Zusammensetzung.

28. Zusammensetzung zur kosmetischen Behandlung der Haare nach einem der vorhergehenden Ansprüche, **dadurch gekennzeichnet, dass** sie mindestens ein Additiv enthält, das ausgewählt ist unter kationischen, anionischen, nichtionischen oder amphoteren Polymeren; natürlichen oder synthetischen anionischen, amphoteren, zwitterionischen, nichtionischen oder kationischen polymeren Verdickungsmitteln, die gegebenenfalls Assoziativpolymere darstellen; nichtpolymeren Verdickungsmitteln; Perlglanzmitteln; Trübungsmitteln; Sonnenfiltern; Parfums; Färbemitteln; organischen oder anorganischen Partikeln; Konservierungsmitteln sowie Mitteln zur Stabilisierung des pH-Werts.

29. Verfahren zur kosmetischen Behandlung der Haare, **dadurch gekennzeichnet, dass** eine Zusammensetzung zur kosmetischen Behandlung nach einem der vorhergehenden Ansprüche auf die Haare aufgebracht wird.

30. Verwendung einer Zusammensetzung zur kosmetischen Behandlung nach einem der Ansprüche 1 bis 28 zur Konditionierung der Haare.

31. Verwendung einer Zusammensetzung zur kosmetischen Behandlung nach Anspruch 30 als Après-Shampoo.

## Claims

1. Cosmetic hair treatment composition, of water-in-oil emulsion type, **characterized in that** it comprises, in a cosmetically acceptable medium, at least one non-volatile non-silicone oil, at least one cationic surfactant, at least one nonionic surfactant and from 0.01% to 10% by weight, relative to the total weight of the composition, of at least one (C₁₂₋₃₀ alkyl)monoglycoside or (C₁₂₋₃₀ alkyl)polyglycoside.

2. Cosmetic hair treatment composition according to Claim 1, **characterized in that** the non-volatile non-silicone oil is a plant oil, an animal oil, a mineral oil, a synthetic oil or a fatty acid ester, or a mixture thereof.

3. Cosmetic hair treatment composition according to Claim 2, **characterized in that** the plant oil is chosen from sweet almond oil, avocado oil, castor oil, olive oil, liquid jojoba wax, sunflower oil, wheatgerm oil, sesame seed oil, groundnut oil, grapeseed oil, soybean oil, rapeseed oil, safflower oil, coconut oil, corn oil, hazelnut oil, palm oil, apricot kernel oil and beauty-leaf oil.

4. Cosmetic hair treatment composition according to Claim 2, **characterized in that** the animal oil is perhydrosqualene.

5. Cosmetic hair treatment composition according to Claim 2, **characterized in that** the mineral oil is chosen from liquid paraffin and liquid petroleum jelly.

6. Cosmetic hair treatment composition according to Claim 2, **characterized in that** the synthetic oil is chosen from squalane, poly(α-olefins), transesterified plant oils and fluoro oils.

7. Cosmetic hair treatment composition according to Claim 2, **characterized in that** the fatty ester is chosen from purcellin oil, isopropyl myristate, isopropyl palmitate, butyl stearate, hexyl laurate, isononyl isononanoate, 2-ethylhexyl palmitate, 2-hexyldecyl laurate, 2-octyldecyl palmitate, 2-octyldodecyl myristate, isostearyl neopentanoate and tridecyl neopentanoate.

8. Cosmetic hair treatment composition according to Claim 1 or 2, **characterized in that** the non-volatile non-silicone oil is chosen from avocado oil, isododecane, isopropyl myristate and liquid jojoba wax.

9. Cosmetic hair treatment composition according to any one of the preceding claims, **characterized in that** it comprises the oil(s) in an amount ranging from 0.1% to 30% by weight, preferably from 1% to 20% by weight and better still from 5% to 15% by weight relative to the total weight of the composition.

10. Cosmetic hair treatment composition according to any one of the preceding claims, **characterized in that** the cationic surfactants are chosen from optionally polyoxyalkylenated primary, secondary or tertiary fatty amine salts and quaternary ammonium salts, and mixtures thereof.

11. Cosmetic hair treatment composition according to Claim 10, **characterized in that** the quaternary ammonium salts are chosen from:
- those corresponding to the general formula (I) below: 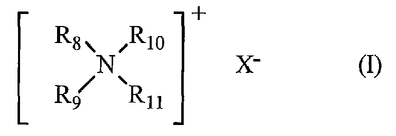 in which the radicals R₈ to R₁₁, which may be identical or different, represent a linear or branched aliphatic radical containing from 1 to 30 carbon atoms, or an aromatic radical; X⁻ is an anion chosen from the group of halides, phosphates, acetates, lactates, (C₂-C₆) alkyl sulfates and alkyl- or alkylaryl-sulfonates;
- quaternary ammonium salts of imidazoline;
- the diquaternary ammonium salts of formula (III): 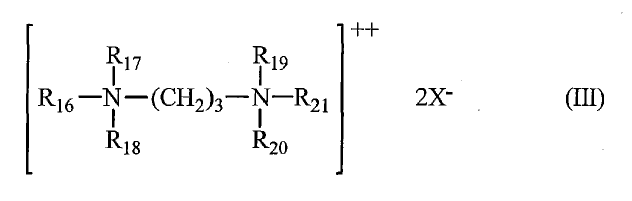 in which R₁₆ denotes an aliphatic radical containing from about 16 to 30 carbon atoms, R₁₇, R₁₈, R₁₉, R₂₀ and R₂₁, which may be identical or different, are chosen from a hydrogen atom and an alkyl radical containing from 1 to 4 carbon atoms, and X⁻ is an anion chosen from the group of halides, acetates, phosphates, nitrates and methyl sulfates;
- quaternary ammonium salts containing at least one ester function.

12. Cosmetic hair treatment composition according to Claim 10 or 11, **characterized in that** the cationic surfactant is chosen from behenyltrimethylammonium chloride and cetyltrimethylammonium chloride.

13. Cosmetic hair treatment composition according to any one of the preceding claims, **characterized in that** it comprises the cationic surfactant(s) in an amount ranging from 0.1% to 20% by weight, preferably from 0.2% to 10% and even more preferably from 0.5% to 8% by weight relative to the total weight of the composition.

14. Cosmetic hair treatment composition according to any one of the preceding claims, **characterized in that** the nonionic surfactant has an HLB ranging from 1.5 to 10.

15. Cosmetic hair treatment composition according to Claim 14, **characterized in that** the HLB ranges from 1.5 to 7.

16. Treatment composition according to any one of the preceding claims, **characterized in that** the nonionic surfactant is chosen from polyethoxylated, polypropoxylated or polyglycerolated alcohols; polyethoxylated, polypropoxylated or polyglycerolated α-diols; polyethoxylated, polypropoxylated or polyglycerolated (C₁₋₂₀)alkylphenols; and polyethoxylated, polypropoxylated or polyglycerolated fatty acids; condensates of ethylene oxide and of propylene oxide with fatty alcohols; polyethoxylated fatty amides; polyglycerolated fatty amides comprising on average from 1 to 5 glycerol groups; ethoxylated fatty acid esters of sorbitan containing from 2 to 30 ethylene oxide units; fatty acid esters of sucrose; fatty acid esters of polyethylene glycol; polyethoxylated plant oils; N-(C₆₋₂₄ alkyl)glucamine derivatives; and amine oxides.

17. Cosmetic hair treatment composition according to claim 16, **characterized in that** the nonionic surfactant is chosen from sorbitan isostearate, polyglyceryl isostearate, methylglucose dioleate and hydrogenated castor oil polyethoxylated with 7 mol of ethylene oxide.

18. Cosmetic hair treatment composition according to any one of the preceding claims, **characterized in that** it comprises the nonionic surfactant(s) in an amount ranging from 0.01% to 10% by weight and preferably from 0.1% to 5% by weight relative to the total weight of the composition.

19. Cosmetic hair treatment composition according to any one of the preceding claims, **characterized in that** the (C₁₂₋₃₀ alkyl)monoglycoside or (C₁₂₋₃₀ alkyl)polyglycoside is chosen from those in which the alkyl group contains from 16 to 24 carbon atoms.

20. Cosmetic hair treatment composition according to any one of the preceding claims, **characterized in that** it comprises the (C₁₂₋₃₀ alkyl) - monoglycoside (s) or (C₁₂₋₃₀ alkyl) polyglycoside(s) in an amount ranging from 0.02% to 5% by weight relative to the total weight of the composition.

21. Cosmetic hair treatment composition according to any one of the preceding claims, **characterized in that** the weight ratio of oil(s)/nonionic surfactant(s) is between 3 and 100 and preferably between 10 and 75.

22. Cosmetic hair treatment composition according to any one of the preceding claims, **characterized in that** it also comprises at least one C₁₄₋₃₀ fatty alcohol.

23. Cosmetic hair treatment composition according to Claim 22, **characterized in that** the C₁₄₋₃₀ fatty alcohol is chosen from myristyl alcohol, cetyl alcohol, stearyl alcohol, arachidyl alcohol, behenyl alcohol and erucyl alcohol.

24. Cosmetic hair treatment composition according to Claim 22 or 23, **characterized in that** it comprises the fatty alcohol(s) in an amount of less than 10% by weight relative to the total weight of the composition.

25. Cosmetic hair treatment composition according to any one of the preceding claims, **characterized in that** the cosmetically acceptable medium comprises water or a mixture of water and of at least one cosmetically acceptable solvent.

26. Cosmetic hair treatment composition according to Claim 25, **characterized in that** the cosmetically acceptable solvent is chosen from C₁-C₄ lower alcohols, polyols, polyol ethers, C₅-C₁₀ alkanes, C₃₋₄ ketones, C₁-C₄ alkyl acetates, dimethoxyethane and diethoxyethane.

27. Cosmetic hair treatment composition according to any one of the preceding claims, **characterized in that** it also comprises at least one silicone oil in an amount of less than 10% by weight relative to the total weight of the composition.

28. Cosmetic hair treatment composition according to any one of the preceding claims, **characterized in that** it comprises at least one additive chosen from cationic, anionic, nonionic or amphoteric polymers; natural or synthetic, anionic, amphoteric, zwitterionic, nonionic or cationic, associative or non-associative polymeric thickeners; non-polymeric thickeners; nacreous agents; opacifiers; sunscreens; fragrances; dyes; organic or mineral particles; preserving agents; and pH stabilizers.

29. Cosmetic hair treatment process, **characterized in that** a cosmetic treatment composition according to any one of the preceding claims is applied to the hair.

30. Use of a cosmetic treatment composition according to any one of Claims 1 to 28 for conditioning the hair.

31. Use of a cosmetic treatment composition according to Claim 30 as a hair conditioner.
